(19) Europäisches Patentamt
European Patent Office
Office européen des brevets

(11) Publication number: 0 040 074 B1

(12) # EUROPEAN PATENT SPECIFICATION

(45) Date of publication of patent specification: 31.07.85

(51) Int. Cl.[4]: C 07 D 221/18, A 61 K 31/485

(21) Application number: 81302069.0

(22) Date of filing: 08.05.81

(54) **N-substituted aporphines, a method of inducing emesis and a method of controlling psychosis utilising the same.**

(30) Priority: 08.05.80 US 147737
08.05.80 US 148179

(43) Date of publication of application:
18.11.81 Bulletin 81/46

(45) Publication of the grant of the patent:
31.07.85 Bulletin 85/31

(84) Designated Contracting States:
CH DE FR GB IT LI

(56) References cited:
DE-A-2 755 511
DE-A-2 758 954
US-A-3 717 643

JOURNAL OF MEDICINAL CHEMISTRY, vol. 23, no. 6, June 1980, Columbus, Ohio, USA, J.L. NEUMEYER et al. "Aporphines 30", pages 594-95
JOURNAL OF MEDICINAL CHEMISTRY, vol. 23, no. 9, September 1980, Columbus, Ohio, USA, J.L. NEUMEYER et al. "Aporphines 31", pages 1008-1013

(73) Proprietor: NORTHEASTERN UNIVERSITY
360 Huntington Avenue
Boston Massachusetts 02115 (US)

(72) Inventor: Neumeyer, John L.
1 Holiday Road
Wayland Massachusetts 01778 (US)

(74) Representative: Warren, Anthony Robert et al
BARON & WARREN 18 South End Kensington
London W8 5BU (GB)

(56) References cited:
CHEMICAL ABSTRACTS, 9th Collective Subject Index, publ. in 1978, Columbus, Ohio, USA, page 13726CS, left-hand column, at the bottom, abstract 78 92509j
JOURNAL OF ORGANIC CHEMISTRY, vol. 42, no. 11, 1977, Columbus, Ohio, USA, F.E. GRANCHELLI et al. "Aporphines 23. Normorphothebaine derivatives: Synthesis of an aporphine nitrogen mustard", pages 2014-17

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to N-substituted dihydroxy and trihydroxynoraporphines and their esters, along with their acid addition salts.

Although emetic drugs that induce vomiting have been used for centuries in the management of patients who have orally ingested poisons, they could have applications other than the removal of such poisons provided they possessed properties not now generally associated with the presently used emetics. For example, it may be necessary for a patient who must undergo emergency surgery to have his stomach emptied quickly and safely before receiving anesthesia. A nontoxic, rapid-acting emetic could be used for this purpose. Moreover, if an emetic can be administered through inhalation, it may be used in place of tear gas and the like to effect control over violent subjects. Such extensions of the use of emetics require that the drug being used is safe, effective at small dosage levels and capable of being administered by routes other than direct oral ingestion or intraveneous injections.

The emetics being currently used are generally either ipecac given orally or apomorphine administered by intravenous injection. Ipecac is, on the average, effective in only about five out of six cases and achieves emesis only after about 15 minutes after taking it. This long reaction time may result in the patient's vomiting after reaching a comatose state. Apomorphine is unstable in solution so that no commercial solution is available. Moreover, in addition to requiring that the solution be made up just prior to injection, apomorphine may cause a depression in the patient's breathing which can be serious if the poison to be removed is a sedative drug.

In addition to apomorphine, N-substituted-norapomorphines of the formula

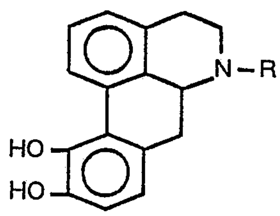

wherein R is lower-alkyl from two to four carbon atoms, 2-chlorobutyl, cyclopropyl, cyclopropylmethyl or 2-phenylethyl, have been proposed as emetics. (See United States Patents 3,717,639 and 3,717,643). However, these norapomorphines are not presently used for this purpose.

Neuroleptic drugs such as phenothiazines, thioxanthenes and butyrophenones, are used in the treatment of psychoses to counteract hallucinations and delusions, and to alleviate psychomotor excitement and assaultiveness. In this role, these drugs reduce dopaminergic activity to the central nervous system by blocking the dopamine receptors, thus preventing the activation of adenylate cyclase identified as the agent responsible for the conversion of adenosine triphosphate (ATP) to cyclic adenosine monophosphate (cyclic AMP), a nucleotide. The generation of cyclic AMP within a brain cell is believed to stimulate the cell. In treating psychoses, e.g., schizophrenia, it has been found that drugs that act as antagonists of the dopamine receptor are effective, indicating that schizophrenia results from an overactivity of dopamine-containing neurons in certain areas of the brain. Thus by controlling such dopamine-containing neurons it is possible to control psychoses.

A number of different neuroleptics are currently prescribed. Typical of the phenothiazines is chlorpromazine hydrochloride

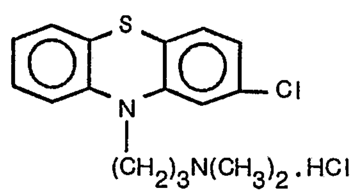

Although this drug is an effective neuroleptic, it must be given as often as three times daily at relatively large dose levels (200 to 800 mg/day); and it has such side effects as sedation, extrapyramidal activity, and the inducing of hypotension.

Chlorprothixene having the formula

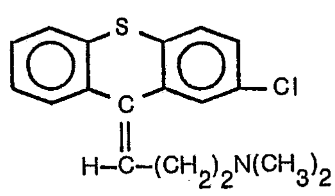

is representative of the thioxanthenes. Like chlorpromazine, it must be given often at a dosage level of from 50 to 400 gms/day and has essentially the same side effects.

The butyrophenones, represented by haloperidol having the formula,

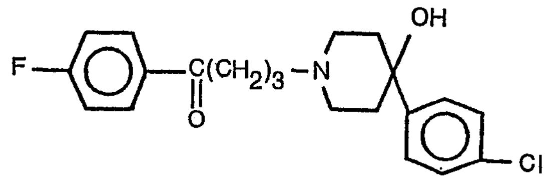

have high neuroleptic potency. Haloperidol produces a high incidence of extrapyramidal reactions; but it has less sedative effect, less prominent antonomic effects and less hypotensive effect than the phenothiazines. Dosage levels for the butyrophenones range from 2 to 6 mg/day.

It is an object of this invention to provide a novel class of N-substituted hydroxyapomorphines, their esters and acid addition salts, some of which exhibit greater emetic efficacy than the emetics now available. It is another object of this invention to provide emetic compounds of the character described which may be administered by one of several routes including orally, by injection, sublingually, topically or by inhalation.

Another object is to provide compounds of the character described which exhibit greater dopaminergic activity than the neuroleptic drugs presently in use. A further object of this invention is to provide such neuroleptic drugs which are longer-acting and hence require administering less frequently than such drugs as chlorpromazine, chlorprothixene or haloperidol. Yet another object is to provide a dopaminergically active compound which inactivates dopamine receptors by binding to them rather than by blocking them.

According to the present invention, there is provided a compound having the formula

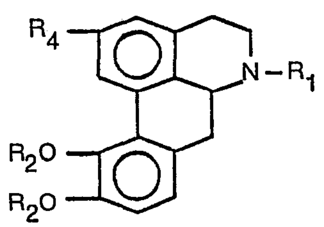

wherein $R_1$ is $C_1$—$C_6$ alkyl, substituted $C_1$—$C_6$ alkyl, $C_3$—$C_8$ cycloalkyl, substituted $C_3$—$C_8$ cycloalkyl, $C_3$—$C_7$ alkenyl, $C_3$—$C_7$ alkynyl, substituted $C_3$—$C_7$ alkynyl, optionally substituted phenyl $C_1$—$C_4$ alkyl, optionally substituted phenyl $C_2$—$C_4$ alkenyl and optionally substituted phenyl $C_2$—$C_4$ alkynyl, $R_2$ is hydrogen or

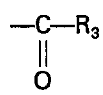

wherein $R_3$ is $C_1$—$C_7$ alkyl, $R_4$ is hydrogen or $R_2O$ and the acid addition salts thereof with the proviso that when $R_4$ is hydrogen, $R_1$ is 2-chloroethyl, and wherein the term "substituted" is defined as $C_1$—$C_6$ alkyl, $C_1$—$C_6$ alkoxy, nitro, $C_1$—$C_6$ alkyl mercapto, methylene dioxy, chloro, bromo, iodo, fluoro or trifluoromethyl.

As will be seen, the three formerly mentioned classes of compounds now in use as neuroleptics are chemically distinguishable from the N-substituted haloalkyl norapomorphines embodying this invention; and therefore it is not apparent that compounds having a structure totally different from the known neuroleptics could be effective in that role. Moreover, apomorphine and its homolog N-propyl-norapomorphine are known as drugs which act by directly stimulating the dopamine receptors. (See G. C. Cotzias, P. S. Papavasieron, E. S. Tolosa, J. S. Mendez and M. Bell-Miduron, *N. Eng. J. Med.* 294:567 (1976)). Thus, whereas apomorphine and N-propylnorapomorphine are recognized as dopamine receptor agonists, the N-substituted haloalkyl norapomorphines embodying this invention are dopamine receptor antagonists.

As will be described below in detail, the compounds embodying this invention are believed to provide dopaminergic activity through binding with the dopamine receptors, thus providing a mechanism different from that exhibited by the currently administered neuroleptic drugs which are blocking agents, and offering advantages over them.

Many of the aporphines embodying this invention exhibit high emetic efficacy at minute dosage levels with no apparent evidence of cardiovascular, central nervous system or respiratory toxicity.

For a fuller understanding of this invention, reference should be had to the following detailed description taken in connection with the accompanying drawings, in which:—

Figure 1 is a plot illustrating the ability of (−)N-(2-chloroethyl)norapomorphine · HCl at two different dosage levels to inhibit circling in apomorphine-challenged mice;

Figure 2 illustrates the effect on rat circling of intrastriatal injections of (−)N-(2-chloroethyl)norapomorphine · HCl prior to treatment with apomorphine;

Figure 3 is a plot illustrating the ability of (−)N-(2-chloroethyl)norapomorphine · HCl at different dosage levels to inhibit climbing in apomorphine-challenged mice;

Figure 4 is a series of bar graphs illustrating the *in vivo* effect of the intrastriatal binding of (N)-(2-chloroethyl)norapomorphine · HCl on the subsequent binding of $^3$H-N-n-propylnorapomorphine;

Figure 5 is a series of bar graphs illustrating the *in vitro* effect of the intrastriatal binding of (N)-(2-chloroethyl)-norapomorphine on the subsequent binding of H-N-n-propylnorapomorphine alone or in the presence of other blocking agents;

Figure 6 is a series of plots illustrating the ability of four different apomorphine-type compounds to reduce the specific binding of $^3$H-N-n-propylnorapomorphine in rat striatal homogenate;

Figure 7 illustrates, in plots, the effect of washing on the binding of (−)N-(2-chloroethyl)norapomorphine · HCl to rat striatal homogenate; and

Figure 8 is an illustrative route for the preparation of the N-substituted norapomorphines embodying the present invention.

An exemplary reaction scheme for synthesizing (−)N-chloroethyl)-10,11-dihydroxynoraporphine · HCl (NCA), the preferred form of the neuroleptic drugs of this invention, will now be described. This synthesis begins with the N-demethylation of codeine to norcodeine, such as by the methods described by M. M. Abdel-Monem and P. S. Portoghese (*J. Med. Chem*: *15,* 208 (1972)) or by K. Rice (*J. Org. Chem*: *40,* 1850 (1975)). Subsequent N-alkylation of the norcodeine with bromoethanol is followed by the rearrangement of the resulting N-hydroxyethylnorcodeine with methanesulfonic acid to give the N-hydroxyethylnorapocodeine, using the procedure of F. E. Granchelli, C. N. Filer, A. H. Soloway and J. L. Neumeyer (*J. Org. Chem*: in Vol, 45, pp. 2275—2278 (1980)). Treatment of the N-hydroxyethylnorapocodeine with 48% hydrogen bromide at 120—135°C under nitrogen for at least 2 hours effects dealkylation to form N-hydroxyethylnorapomorphine hydrobromide which is then converted to the hydrogen chloride salt by neutralization of the reaction mixture with ammonium hydroxide, followed by extraction and then treatment of the resulting free base with ethereal HCl. Finally, treatment of this acid addition salt with thionyl chloride and acetonitrile at room temperature yields the desired (−)N-chloroethyl)-10,11-dihydroxynoraporphine after neutralization of the evaporated reaction mixture with ammonium hydroxide. The hydrogen chloride salt is prepared from an ethereal HCl extract, mp 173—178°C.

NMR and UV spectra as well as elemental analysis are used to establish the structure of the compound. NMR of the free base (MeOH-$d_4$) δ 2.33—4.40 (broad signals, 13, H at C-4, C-5, C-6a, C-7, $NCH_2CH_2Cl$ and phenolic OH) 6.53—7.40 (m, 4, aromatic), 8.1—8.37 (dd, 1, aromatic H at C-1); MS, m/e 315 ($M^+$), 266 (base); $UV_{max}$ (EtOH) 275 nm, (log ε, 4.169); $[\alpha]^{28}_{Hg}546$ −35°. Anal. Calcd for $C_{18}H_{23}NO_3Cl$ (MW 336.82): C, 55.68; H, 5.97; N, 3.60. Found: C, 55.96; H, 5.34; N, 3.59.

Using well-known techniques the free base and acid addition salts may thus be converted from one form to the other and one acid addition salt may be converted to another by regenerating the free base form and acidifying it.

Another of the N-substituted noraporphines of this invention may be prepared from the readily available opium alkaloid thebaine by the synthesis scheme illustrated in Figure 8.

In this synthesis scheme, thebaine is N-demethylated to northebaine using diethylazodicarboxylate according to the method disclosed in British Patent 1,124,441 (August 21, 1968). The rearrangement of the northebaine to normorphothebaine is accomplished with concentrated HCl in a sealed pressure bottle on a steam bath for 2.5 hours according to a published procedure (F. E. Granchelli, A. H. Soloway, J. L. Neumeyer and C. N. Filer, *J. Org. Chem*: *42,* 2014 (1977).) Formation of the N-$R_1$-normorphothebaine · HCl is carried out in the presence of an acid acceptor (e.g., $Na_2CO_3$ or $NaHCO_3$) using the appropriate $R_1$ iodide or $R_1$ bromide in a suitable solvent system at about 100°C under nitrogen for 16 to 24 hours. After isolation and purification of the product N-$R_1$-normorphothebaine, it is heated with 48% HBr at 130—135°C under nitrogen for three to four hours to form the HBr acid addition salt of the desired N-$R_1$-2,10,11-trihydroxynoraporphine. Neutralization of the acid addition salt with $NH_4OH$ forms the free base which then may, if required, be converted to the HCl salt by treatment with ethereal HCl.

The ester derivatives wherein $R_2$ is

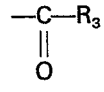

and $R_3$ is lower alkyl are conveniently prepared by reacting the appropriate N-$R_1$-2,10,11-trihydroxynoraporphine · HBr or free base with an appropriate acyl halide to give the substituted triacyl noraporphine.

Appropriate acid addition salts are those derived from such diverse acids as formic acid, acetic acid, isobutyric acid, alpha-mercaptopropionic acid, malic acid, fumaric acid, succinic acid, succinamic acid, tartaric acid, citric acid, lactic acid, benzoic acid, 4-methoxybenzoic acid, phthalic acid, anthranilic acid, 1-naphthalenecarboxylic acid, cinnamic acid, cyclohexanecarboxylic acid, mandelic acid, tropic acid, crotonic acid, acetylene dicarboxylic acid, sorbic acid, 2-furancarboxylic acid, cholic acid, pyrenecarboxylic acid, 2-pyridinecarboxylic acid, 3-indoleacetic acid, quinic acid, sulfamic acid, methanesulfonic acid, isethionic acid, benzenesulfonic acid, p-toluenesulfonic acid, benzenesulfinic acid, butylarsonic acid, diethylphosphinic acid, p-aminophenylarsinic acid, phenylstibnic acid, phenylphosphinous acid, methylphosphinic acid, phenylphosphinic acid, hydrofluoric acid, hydrochloric acid, hydrobromic acid, hydriodic

acid, perchloric acid, nitric acid, sulfuric acid, phosphoric acid, hydrocyanic acid, phosphotungstic acid, molybdic acid, phosphomolybdic acid, pyrophosphoric acid, arsenic acid, picric acid, picrolonic acid, barbituric acid, boron trifluoride, and the like.

As used herein, the term "$C_1$—$C_6$ alkyl" means saturated monovalent aliphatic radicals, including straight and branched-chain radicals, as illustrated by, but not limited to methyl, ethyl, propyl, isopropyl, butyl, sec.-butyl, amyl, or hexyl.

As used herein, the term "$C_3$—$C_7$ alkenyl" means monovalent, aliphatic radicals which contain at least one double bond, and are either straight or branched-chain, as illustrated by, but not limited to 1-(2-propenyl), 1-(3-methyl-2-propenyl), 1-(1,3-dimethyl-2-propenyl), or 1-(2-hexenyl).

As used herein, the term "$C_3$—$C_7$ alkynyl" means monovalent, aliphatic radicals which contain at least one triple bond, and are either straight or branched, as illustrated by, but not limited to 1-(2-propynyl), 1-(1-methyl-2-propynyl), or 1-(2-heptynl).

As used herein, the term "substituted" is intended to mean those alkyl, cycloalkyl, alkenyl and alkynyl groups bearing substituents such selected from the group $C_1$—$C_6$ alkyl, $C_1$—$C_6$ alkoxy, nitro, $C_1$—$C_6$ alkylmercapto, methylenedioxy, halo and trifluoromethyl.

As used herein, the term "cycloalkyl" means cyclic, saturated aliphatic radicals of from three to eight ring carbon atoms, as illustrated by, but not limited to cyclopropyl, cyclobutyl, 2-methylcyclobutyl, cyclohexyl, 4-methylcyclohexyl, or cyclooctyl.

As used herein, the terms "phenyl-$C_1$—$C_4$-alkyl," "phenyl-$C_2$—$C_4$-alkenyl," and "phenyl-$C_2$—$C_4$-alkynyl" mean monovalent radicals consisting of a phenyl nucleus bonded to the rest of the molecule through, respectively, a divalent alkylene radical, as illustrated by, but not limited to methylene, 1,1-ethylene, 1,2-ethylene, 1,3-propylene, 1,2-propylene, or 1,4-butylene; or through a divalent alkynylene or alkenylene radical, as illustrated by, but not limited to 1,2-ethynylene, 1,3-propynylene, 1,3-(butynylene), and the like. Moreover the benzene ring of such phenyl-$C_1$—$C_4$-alkyl, phenyl-$C_2$—$C_4$-alkenyl, and phenyl-$C_2$—$C_4$-alkynyl radicals can be substituted by one or more substituents selected from the group consisting of $C_1$—$C_6$-alkyl, $C_1$—$C_6$-alkoxy, halo(chloro, bromo, iodo, or fluoro), nitro, $C_1$—$C_6$-alkylmercapto, methylenedioxy, and trifluoromethyl.

The following examples of the preparation of examplary compounds, which are meant to be illustrative and not limiting, are provided as a further description of the invention.

Examples 1—4

Normorphothebaine hydrochloride was prepared from thebaine using the synthesis scheme outlined above and according to the teaching of British Patent 1,124,441 and Granchelli et al. Four N-substituted normorphothebaines were then made using the halides and solvent systems detailed below in Table 1.

TABLE 1
Preparation of four N-substituted normorphothebaines

| | Example No. | | | |
|---|---|---|---|---|
| Item | 1 | 2 | 3 | 4 |
| N-Substituent<br>$R_1$ | ethyl<br>$—C_2H_5$ | n-propyl<br>n-$C_3H_7$ | allyl<br>$—CH_2CH{=}CH_2$ | propargyl<br>$—CH_2C{\equiv}CH$ |
| $R_1$ halide | $C_2H_5I$ | n-$C_3H_7I$ | $CH_2{=}CHCH_2Br$ | $CH{\equiv}CCH_2Br$ |
| Solvent system | 5% $CH_3OH$ in $CHCl_3$ | 5% $CH_3OH$ in $CHCl_3$ | 3% $CH_3OH$ in $CHCl_3$ | $CH_3OH$:$Et_2O$:$CHCl_3$ (3:32:65) |
| $R_f$ values* | 0.19 | 0.24 | 0.30 | 0.34 |
| Yield, % | 65 | 51 | 54 | 75 |
| Form | HCl salt | HCl salt | HCl salt | free base |
| M.P., °C | 209—211 | 214—216 | 204—206 | 175—176 |

* (5% $CH_3OH$ in $CHCl_3$)

In these syntheses a mixture of 5.10 grams (0.016 mole) of the normorphothebaine hydrochloride, 5.0 grams (0.06 mole) of $NaHCO_3$, 0.02 mole of the $R_1$ halide in 130 milliliters of acetonitrile was heated at 100°C under nitrogen for 16 hours. In Examples 3 and 4 where the $R_1$ halide was a bromide, 0.014 mole of potassium iodide was added to the mixture with the bromide. The resulting reaction mixture was cooled and filtered, and the filtrate was evaporated to a residue. The residue was then treated with 100 milliliters of

chloroform and filtered. The filtrate, concentrated to about 5 milliliters solution, was then chromatographed on silica gel (1:30) packed and eluted with a mixed solvent (5% $CH_3OH$ in $CHCl_3$) to give the desired compound in a yield and with a melting point given in Table 1.

Examples 5—7

Separate mixtures of 1.8 mmoles of the N-ethyl-, N-n-propyl- and N-allylnormorphothebaines of Examples 1—3, respectively, with 18 milliliters of 48% HBr were heated at 130 to 135°C under nitrogen for three to four hours. The reaction mixtures were cooled and then evaporated to a dry residue in vacuo. The residues were recrystallized from methanol/ethyl ether to give off-white solids in the yields and with the melting points given below in Table 2.

TABLE 2
N-Substituted-2,10,11-trihydroxynorapophine HBr's

| Item | Example No. 5 | 6 | 7 |
|---|---|---|---|
| N-substituent | ethyl | n-propyl | allyl |
| $R_1$ | $—C_2H_5$ | $n\text{-}C_3H_7$ | $—CH_2CH{=}CH_2$ |
| Yield, % | 85 | 70 | 89 |
| M.P., °C | 238—241 | 203—206 | 208—210 |

Example 8

(−)N-(2′-Bromoallyl)-2,10,11-trihydroxynoraporphine hydrochloride

A mixture of 0.30 gram (0.93 mmole) of the N-propargylnormorphothebaine of Example 4 in 10 milliliters of 48% HBr was heated and evaporated in the same manner as described for the preparation of Examples 5—7 to give a dry residue (0.35 gram). Further purification of the product was carried out through the preparation of its triacetoxy derivative followed by subsequent hydrolysis. The residue was diluted with 10 milliliters of trifluoroacetic acid and added dropwise with 4.0 milliliters of acetyl bromide to form a complete solution.

After the initial exothermic reaction subsided, the reaction mixture was heated at 90°C under nitrogen for 2 hours and then evaporated to a residue. The residue was taken up with 50 milliliters of chloroform, washed with 30 milliliters of 3% $NaHCO_3$, brine, dried, and concentrated to approximately a 2 milliliter solution. This solution was then chromatographed on silica gel packed and eluted with chloroform/ethyl ether to give a pure product which showed $R_f$ of 0.32 (in chloroform) on TLC. The triacetoxy derivative underwent alcoholysis after being treated with a mixture of 25 milliliters of absolute methanol and 12.5 milliliters of ethereal HCl for 16 hours. The reaction mixture was evaporated to a residue which was recrystallized from methanol/ethyl ether to give an off-white solid (120 mg, 38%), mp 180—184°C. TLC showed $R_f$ of 0.40 in methanol/chloroform (1:6).

In a manner similar to that detailed in Examples 1—4, the N-$R_1$-normorphothebaines wherein $R_1$ is phenyl lower alkyl, phenyl lower alkenyl or phenyl lower alkynyl are advantageously prepared by reaction, in the presence of an acid acceptor, of the normorphothebaine · HCl with an appropriate phenyl lower alkyl halide, phenyl lower alkenyl halide or phenyl lower alkynyl halide.

Example 9

(−)N-n-Propyl-2,10,11-triacetoxynoraporphine hydrochloride hydrate.

A mixture of N-n-propyl-2,10,11-trihydroxynoraporphine hydrobromide (1.16 grams, 0.0029 mole) prepared as in Example 6 and trifluoroacetic acid (10 milliliters) was added with acetyl bromide (5.0 milliliters) to result in complete solution. The reaction mixture was heated at 90°C under nitrogen for four hours and evaporated to a viscous residue. The residue was taken up with 50 milliliters of chloroform and the resulting solution was washed with 3% $NaHCO_3$ (30 milliliters), brine, dried and evaporated to give an oily residue. This residue was chromatographed on a silica gel column packed and eluted with chloroform/ethyl ether (1:1) to give the pure desired product ($R_f$=0.25 in chloroform).

The hydrochloride salt was prepared by dissolving the free base in 200 milliliters of anhydrous ether and the solution was treated with an excess of ethereal HCl. Filtration of the mixture gave a white solid (1.09 grams, 80%) with m.p. 179—183°C, and a structure confirmed by NMR and infrared spectra. Anal. Calcd. for $C_{25}H_{27}NO_6 \cdot HCl \cdot 1 \cdot 5H_2O$ (MW 500.97): C, 59.93; H, 6.23; N, 2.80. Found: C, 59.81; H, 6.34; N, 2.81.

Compounds of this invention have been found to be very effective emetics. Emetic efficacy was measured in alert dogs with three particulate (15 µm spheres) and three soluble markers (phenol red theophylline and tobramycin) instilled 5, 25 and 55 minutes before rapid intravenous injection of the

emetic. Apomorphine was used as a control emetic. Measurements included time interval to first emesis and recovery in the vomitus of the particulate and soluble markers.

The (−)N-n-propyl-2,10,11-trihydroxynoraporphine · HBr of Example 6, made up in a solution of physiological saline or other suitable vehicle and administered in doses ranging between 0.005 and 1 mg/kg body weight, produced emesis in conscious dogs within two minutes of an intraveneous injection. Even at the high dosage level of 1 mg/kg there was no evidence of cardiovascular, central nervous system or respiratory toxcity. At a dosage level of 0.1 mg/kg the (−)N-n-propyl-2,10,11-trihydroxy-norapomorphine · HBr was found to be 50 times more potent than apomorphine. The (−)N-n-propyl-2,10,11-triacetoxynoraporphine · HCl of Example 9 was determined by the above-described tests to be 43 times more potent as an emetic than apomorphine.

It was also found that these two noraporphines of this invention were effective emetics when inhaled in solutions in an appropriate solvent system or in powder form.

Example 10
(−)N-(2-hydroxyethyl)norapomorphine · HBr

A mixture of N-hydroxyethylnorapocodeine (2.51 grams, 8.06 mmole), prepared by the synthesis route described above, and 50 milliliters of 48% HBr was heated at 130°C under nitrogen for four hours. The reaction mixture was cooled and then evaporated to a dry residue in vacuo. The resulting residue was recrystallized from methanol/ethyl ether to give an offwhite solid (2.21 grams, 72%), mp 235—238°C. TLC (precoated silica gel on polyethylene terephthalate foil) showed $R_f$ of 0.24 in ethanol/chloroform 1/9 volume).

Example 11
(−)N-(2-Chloroethyl)norapomorphine · HCl.

1.0 milliliter of thionyl chloride was added dropwise to a mixture of (−)N-(2-hydroxyethyl)nora-pomorphine (0.50 gram free base, 1.7 mmole) in 10 milliliters of dry acetonitrile. The resulting solution was stirred at room temperature for 24 hours, evaporated to a residue in vacuo, diluted with 25 milliliters of water, neutralized with ammonium hydroxide and extracted with chloroform (3×25 milliliters). The combined extracts were dried ($Na_2SO_4$) and evaporated to a residue which was triturated with 50 milliliters of ethyl ether. The ethereal filtrate was treated with excess ethereal HCl to give a light green solid (0.38 gram, 64%), mp 173—178° dec. TLC showed $R_f$ of 0.23 in ethyl acetate.

The (−)N-(2-chloroethyl)norapomorphine · HCl of Example 11 may be converted to the ester form wherein $R_2$ is

$$-\underset{\underset{O}{\|}}{C}-R_3$$

by heating with the appropriate $R_3COCl$. Thus diacetyl (−)N-(2-chloroethyl)apomorphine may be formed by heating (−)N-(2-chloroethyl)norapomorphine · HCl with acetyl chloride.

Apomorphine-induced behavioral characteristics in the rodent are considered to involve dopamine agonist action on striatal and mesolimbic dopamine receptors. Therefore, the ability of a drug to inhibit such apomorphine-induced behavior is considered a measure of the effectiveness of the drug to antagonize dopamine receptors and hence a measure of its dopaminergic activity. Among such induced behavioral characteristics are circling, climbing and stereotypy.

The observed ability of the N-substituted haloalkyl norapomorphines of this invention to inhibit striatal and possibly mesolimbic dopamine function over a period of days in contrast to a few hours for the phenothiazines and butyrophenones suggests that they bind irreversibly to the receptor rather than provide a temporary blocking action. Biochemical assays, reported below, show that the compounds of this invention are able to inhibit $^3$H-NPA ($^3$H-N-n-propylnorapomorphine) binding to rat striatal tissue. It is submitted that such inhibition is the result of the binding of the N-substituted haloalkyl norapomorphines to the dopamine receptor.

Male albino mice in the weight range of 35—40 grams were used in the determination of circling behavior. The mice were prepared for circling experiments using standard stereotaxic techniques to place unilateral electrolesions in the right caudate-putamen (1.0 mm anterior to bregma, 2.0 mm lateral and 3.5 mm below the skull surface, 1.5 mA/15 s). Fourteen days after the surgery the mice were challenged with 0.5 mg/kg apomorphine administered subcutaneously. Those mice circling less than 6 revolutions every 2 minutes were excluded from subsequent studies. Circling behavior was dose-dependent, 0.25—1 mg/kg apomorphine causing from 2—10 revolutions every 12 minutes. A dosage of 0.5 mg/kg apomorphine was selected for use in the drug interaction studies described. At this dosage level of subcutaneously administered apomorphine, the test animals circled from 6 to 8 revolutions/2 minutes.

The unilateral striatectomized mice antagonized by apomorphine were then given, by subcutaneous injection, doses of 1.0, 2.0 or 4.0 mg/kg of the (−)N-(2-chloroethyl)norapomorphine · HCl prepared in Example 11 and made up in a N,N-dimethylformamide/water solution. Circling in an open field was observed to determine the degree to which the circling was inhibited 4 hours and 2, 5 and 7 days after

administration. At the lowest dosage level of 1.0 mg/kg, essentially no inhibition was noted; but as shown in the plots of Figure 1, at the higher dosage levels, the reduction in circling was still apparent after 2 days and at the highest dosage level it was as much as 39% after 5 days. Thus these data clearly show that ipsilateral circling behavior induced in unilateral striatectomized mice by apomorphine, one of the most valuable models for indicating change in striatal dopamine function, could be abolished by the peripheral administration of the (−)N-(2-chloroethyl)norapomorphine · HCl presumably by an action on striatal dopamine receptors in the "intact" hemisphere.

A further behavioral model utilized rats (male, Sprague-Dawley, 300±25 g). The (−)N-(2-chloroethyl)norapomorphine · HCl was injected unilaterally into the striatum of the rat and then it was determined whether a "dopamine blockade" had been achieved by subsequently challenging with apomorphine and measuring any circling induced. Bilateral guide cannulae were chronically implanted in the rats, using standard stereotaxic techniques, to allow drug/vehicle injection at the center of the caudate-putamen complex (Ant. 8.0, Lat.+3.0, Vert. +1.5, De Groot, 1959). Fourteen days after cannulation, the animals were manually restrained and the drug in a solvent vehicle was injected in a volume of 4 μ liters (1 μ liter/min) into the right and left striata, respectively. The 4 μ liter contained dosages of 10 μg or 40 μg of the drug. Any body asymmetry or circling movements resulting from the intracerebral injections were noted, and subsequently any circling behaviour (measured in an open field as revolutions/minute) to challenge with 0.5 mg/kg subcutaneously administered apomorphine was measured on the second, fifth, seventh and fourteenth days following intrastriatal administration.

The unilateral injection of 10 and 40 μg of the (−)N-(2-chloroethyl)norapomorphine · HCl into the caudate-putamen induced ipsilateral asymmetry/circling movements within an hour of administration. The resting asymmetry was particularly evident when the animals were disturbed and was also noted two and five days after the injection of the (−)N-(2-chloroethyl)norapomorphine. By the seventh day the ipsilateral asymmetry was variable and was not apparent by the fourteenth day. The subcutaneous administration of apomorphine (0.5 mg/kg) enhanced the ipsilateral asymmetry and precipitated a clear circling behavior at the 40 μg dose of the drug four hours and two days, and to a lesser extent 5 days after its administration as shown in Figure 2.

Thus it appears logical to conclude that the intrastriatal injection of the (−)N-(2-chloroethyl)norapomorphine in the rat caused a blockade of dopamine receptors at the site of injection since, if the injection was made into one hemisphere only, peripherally administered apomorphine caused rats to circle ipsilaterally to that side, which would again indicate a limitation of action to the "intact" hemisphere. Again, as in the circling experiments with the mice, it was apparent that the dopamine inhibitory actions of the drug endured for 2 to 4 days. The recovery of the behavioral response possibly reflects the resynthesis of dopamine receptors.

Climbing behavior was measured by placing mice in individual cages (20×15×15 cm) lined with wire mesh. A "climbing index", i.e., the percentage of time spent climbing during the 30-minute period following the first climb, was determined. Previous experience had indicated that this climbing index is reduced by dopamine antagonists except where the interacting drug causes a nonspecific sedation or muscular hypotonia. Apomorphine administered subcutaneously at a dosage level of 1 mg/kg caused intense and consistent climbing and provided a basis for the assessment of the effects of potential antagonists. At this dosage the climbing index was approximately 60% which serves as a basis for this assessment.

In the climbing tests, the mice were pretreated with the drug to be evaluated as a dopamine atagonist by giving them intraperitoneal injections of 1, 10 or 20 mg/kg doses of the drug and then subjecting them to a subcutaneous injection of 1.0 mg/kg dose of apomorphine one hour, two days, 5 days or 7 days after the injection of the drug. Taking a climbing index of 60% for apomorphine, it was found that (−)N(2-chloroethyl)norapomorphine · HCl (NCA) reduced this index when given in 10 mg/kg doses, and greatly reduced it when given in 20 mg/kg doses. This effect was noted up to between four and five days after administration of the NCA. Responses to the apomorphine approached control levels five days after the pretreatment. Attempts to overcome the inhibition observed four hours to two days after administration of (−)N-(2-chloroethyl)norapomorphine · HCl by increasing the dose of apomorphine were made impractical by the development of stereotyped biting at the larger doses which reduced the climbing response per se.

In contrast to this ability of the (−)N-(2-chloroethyl)norapomorphine to materially reduce the climbing index, four-hour pretreatments with (−)N-(2-chloroethyl)norapocodiene · HCl or 6-[2-bis(2-chloroethyl)amino]acetyl-11-acetoxy-2-hydroxy-10-methoxy-apoporphine hemihydrate or 90-minute pretreatment with (−)N-(2-hydroxyethyl)norapomorphine · HCl, each given as 10 mg/kg subcutaneous injections, failed to modify apomorphine climbing.

Pretreatments with any of these drugs including NCA in doses of 10 mg/kg prior to apomorphine injections of 1 mg/kg failed to modify the submaximal stereotypic effect (repetitive sniffing, periodic biting and continuous biting) of the apomorphine.

In addition to the in vivo testing of the (−)N-(2-chloroethyl)norapomorphine · HCl, in vitro evaluations were made to determine the extent to which this drug could inhibit the activation of adenylate cyclase to stimulate the production of cyclic adenosine monophosphate (cAMP) in rat striatal homogenates. (−)N-(2-chloroethyl)norapomorphine · HCl (NCA) at three levels (10, 30 and 75 μ moles) was incubated for

10 minutes at 37°C with homogenates of rat corpus striatum in a physiologic buffer and then washed free of the drug. Washed tissue, including one specimen without NCA as a control, was then incubated with zero or 50 µM of dopamine in the presence of an excess of adenosine triphosphate for 2.5 minutes at 37°C and the level of cyclic adenosine monophosphate in the incubation mixture with and without dopamine was assayed by a protein binding method. The increase in cyclic adenosine monophosphate levels due to the dopamine was estimated for all conditions. The ability of the (−)N-(2-chloroethyl)norapomorphine · HCl to inhibit the actuation of adenylate cyclase is shown in Table 1.

TABLE 3

Ability of (−)N-(2-chloroethyl)norapomorphine · HCl (NCA) To inhibit activation of dopamine-sensitive Adenylate cyclase in rat striatal homogenates

| NCA (µM) | Stimulation of cAMP production (%) | Inhibition of adenylate cyclase (%) |
|---|---|---|
| 0 | 100 | 0 |
| 10 | 84 | 16 |
| 30 | 44 | 56 |
| 75 | 8 | 92 |

These effects of the (−)N-(2-chloroethyl)norapomorphine · HCl on the dopamine-sensitive adenylate cyclase are similar to those of phenoxybenzamine, a alkylating drug previously found to exert an irreversible blocking action of dopamine-sensitive adenylate cyclase. (See K. G. Walton, P. Liepmann and R. J. Baldessarini, Eur. J. Pharmacol. 52: 231 (1978).)

The ability of the (−)N-(2-chloroethyl)norapomorphine · HCl to serve as a dopaminergic agent over a prolonged period, i.e., at least five days, leads to the postulation that this drug may bind chemically to the dopamine receptors rather than act as a blocking agent as do the phenathiazines, thioxanthenes and butyrophenones. This postulation was borne out by measuring the effect of the intrastriatal binding of the drug on the subsequent binding of $^3$H-N-n-propylnorapomorphine ($^3$H-NPA), a known dopamine agonist, to the striatal tissue.

In one set of experiments three types of striatal tissue were examined: (1) that taken from untreated rats as a control; (2) that taken from rats receiving 40 µg of the NCA in 4 µ liters of a solvent vehicle at a rate of 1 µ liter/minute in the left hemisphere; and (3) that taken from the right hemisphere subjected to 4 µ liters (1 µ liter/minute) of the solvent vehicle. Animals were sacrificed on days 2, 7 and 14—16 days after the intrastriatal injection; and the striata were dissected out over ice and homogenized (100 vols, w/v) in 50 mM ice cold trihydroxymethyl amine · HCl buffer (pH 7.4 at 25°C) with a Polytron homogenizer. The homogenate was centrifuged twice (for 10 minutes at 50,000 g) at 4°C with resuspension in fresh buffer. Final resuspension (100 vols w/v) was in trihydroxymethyl amine buffer (pH 7.4 at 37°C) containing 0.1% ascorbic acid, 12.5 µM nialamide and 5 mM sodium ethylenediaminetetraacetate. Each assay tube contained 5 mg wet weight striatal tissue (equivalent to approximately 275 µ gram protein, in a total volume of 1.1 milliliter. After incubation (15 minutes at 37°C) with $^3$H-NPA (61 Ci/m mol, New England Nuclear), the samples were rapidly filtered under vacuum over Whatman GF/B filters and rinsed rapidly with 2×5 milliliters ice cold trihydroxymethyl amine · HCl buffer; and the bound radioactivity was determined. Specific binding was defined as the difference between $^3$H-NPA binding in the absence and presence of 10 µM of 2-amino-6-7-dihydroxytetralin (a known dopamine agonist) and under optimal conditions accounted for approximately 75% of $^3$H-NPA less than 2.0 nM, it was found that $^3$H-NPA binding sites had a density of approximately 180 fmol/mg protein.

The results of these measurements of in vivo binding are given in Figure 5, from which it will be seen that the density of the $^3$H-NPA binding sites was essentially reduced to one-half two days after injection of the NCA. Thus these data indicate intrastriatal NCA can significantly reduce $^3$H-NPA binding and that this biochemical change follows the behavioral effects which essentially reflected "striatectomy" by NCA. Thus, on day 2, when animals showed not only an active ipsilateral circling when challenged with apomorphine but also exhibited a resting ipsilateral asymmetry (each identical to effects one would expect from striatectomy or striatal dopamine receptor blockade) the number of binding sites for the dopamine agonist $^3$H-NPA was reduced by approximately 50%. As the behavioral effects of NCA disappeared on days 7 and 14, so the number of sites available for the binding of $^3$H-NPA returned to control values. That the reduction in $^3$H-NPA binding reflects a change in receptor numbers rather than a change in affinity for the receptor sites is indicated by the constancy of values (0.88—1.12 nM) for the inhibition constant, $K_D$, for binding to striatal tissue both from control animals and those treated for various times with NCA.

The ability of (−)N-(2-chloroethyl)norapomorphine · HCl (NCA) to inhibit the binding of $^3$H-NPA was further established in in vitro experiments which facilitated an examination of the doses and conditions

under which NCA could inhibit dopamine mechanisms. Incubation tubes containing rat striatal homogenate were preincubated for 2.5 minutes at 37°C with NCA at five concentrations ($3\times10^{-5}$ to $3\times10^{-7}$ M); with 2-amino-6,7-dihydroxytetralin (ADTN) with ADTN and NCA ($3\times10^{-5}$ M); with propanolol ($1\times10^{-6}$ M) and NCA ($3\times10^{-5}$ M) prior to the addition of 0.5 nM $^3$H-NPA for incubation at 37°C for 15 minutes. Membranes were collected by filtration and measurements were made using the same technique described above. The data obtained in terms of percent of $^3$H-NPA binding are plotted in Figure 4. From these data it will be seen that NCA at $1.8\times10^{-6}$ M caused a 50% inhibition of $^3$H-NPA binding. Moreover, this inhibition was also apparent in the presence of high concentrations of phentolamine (an α-blocking agent) and propanolol (a β-blocking agent which could be expected to hinder occupancy of other catecholamine receptors by either $^3$H-NPA or NCA. Furthermore, the inhibition of "specific" $^3$H-NPA binding by ADTN could not be further increased by NCA, indicating that the ability of the NCA to prevent $^3$H-NPA binding does not occur at "nonspecific" sites. Finally, the prevention of $^3$H-NPA binding by NCA ($10^{-5}$ M) was not reversed by repeated washing.

Using a similar technique and 10 μM of ADTN to first assess the "specific" binding of $^3$H-NPA to rat striatal homogenate, it was determined that the $^3$H-NPA bound to the striatal membranes with an inhibition constant, $K_D$, of 1.0 nM and $B_{max}$ of 190 fmol/mg protein. With this base to work with, the abilities of four aporphine derivatives to prevent the specific binding of $^3$H-NPA were determined and the results are plotted in Figure 6. From this plot it will be seen that the NHA is a more potent binding agent at low concentrations than NCA. However, as will be seen from Figure 7, when the incubated membranes with NCA ($5\times10^{-6}$ M) or with NHA ($5\times10^{-7}$ M), both of which prevented the binding of 0.125 nM $^3$H-NPA, were washed, only the inhibition provided by the NCA was not reversed. Thus it alone was capable of sustained binding to the rat striatal homogenate protein.

The in vivo data show that the (−)N-(2-chloroethyl)norapomorphine · HCl, when administered either peripherally or intrastriatally, can effect behavioral changes indicative of changes at the receptor level associated with dopamine receptor blockade. The in vitro data further show that such inhibition is the result of binding to the receptor, a fact which may explain the prolonged action of the drug. These two properties offer the possiblity of providing an improved neuroleptic drug.

**Claims**

1. A compound of the formula

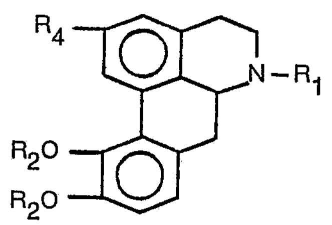

wherein $R_1$ is $C_1$—$C_6$ alkyl, substituted $C_1$—$C_6$ alkyl, $C_3$—$C_8$ cycloalkyl, substituted $C_3$—$C_8$ cycloalkyl, $C_3$—$C_7$ alkenyl, $C_3$—$C_7$ alkynyl, substituted $C_3$—$C_7$ alkynyl, optionally substituted phenyl $C_1$—$C_4$ alkyl, optionally substituted phenyl $C_2$—$C_4$ alkenyl and optionally substituted phenyl $C_2$—$C_4$ alkynyl, $R_2$ is hydrogen or

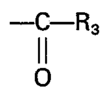

wherein $R_3$ is $C_1$—$C_7$ alkyl, $R_4$ is hydrogen or $R_2O$ and the acid addition salts thereof with the proviso that when $R_4$ is hydrogen, $R_1$ is 2-chloroethyl, and wherein the term "substituted" is defined as $C_1$—$C_6$ alkyl, $C_1$—$C_6$ alkoxy, nitro, $C_1$—$C_6$ alkyl mercapto, methylene dioxy, chloro, bromo, iodo, fluoro or trifluoromethyl.

2. A compound in accordance with claim 1, wherein $R_1$ is ethyl and said compound is (−)N-ethyl-2,10,11-trihydroxynoraporphine.

3. A compound in accordance with claim 1, wherein $R_1$ is n-propyl and said compound is (−)N-n-propyl-2,10,11-trihydroxynoraporphine.

4. A compound in accordance with claim 1, wherein $R_3$ is methyl and said compound is (−)N-n-propyl-2,10,11-triacetoxynoraporphine.

5. A compound in accordance with claim 1, wherein $R_1$ is allyl and said compound is (−)N-allyl-2,10,11-trihydroxynoraporphine.

6. A compound in accordance with claim 1, wherein $R_1$ is 2'-bromoallyl and said compound is N-(2'-bromoallyl)-2,10,11-trihydroxynoraporphine.

7. The compound according to claim 1, wherein $R_1$ is 2-chloroethyl, $R_2$ is hydrogen or

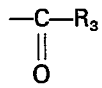

$R_3$ is $C_1$—$C_6$ alkyl, $R_4$ is hydrogen and the acid addition salts thereof.

8. A compound according to claim 7 that is (−)N-(2-chloroethyl)norapomorphine.

**Patentansprüche**

1. Verbindung der Formel

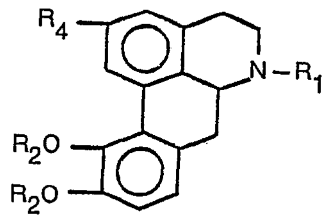

worin $R_1$ eine $C_1$—$C_6$-Alkylgruppe, eine substituierte $C_1$—$C_6$-Alkylgruppe, eine $C_3$—$C_8$-Cycloalkylgruppe, eine substituierte $C_3$—$C_8$-Cycloalkylgruppe, eine $C_3$—$C_7$-Alkenylgruppe, eine $C_3$—$C_7$-Alkinylgruppe, eine substituierte $C_3$—$C_7$-Alkinylgruppe, eine gegebenenfalls substituierte Phenyl-$C_1$—$C_4$-Alkylgruppe, eine gegebenenfalls substituierte Phenyl-$C_2$—$C_4$-Alkenylgruppe und eine gegebenenfalls substituierte Phenyl-$C_2$—$C_4$-Alkinylgruppe, $R_2$ Wasserstoff oder die Gruppe

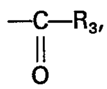

worin $R_3$ eine $C_1$—$C_7$-Alkylgruppe ist, $R_4$ Wasserstoff oder eine Gruppe $R_2O$ bedeuten und deren Säureadditonssalze mit der Maßgabe, daß, falls $R_4$ Wasserstoff ist, $R_1$ eine 2-Chlorethylgruppe ist und worin der Ausdruck "substituiert" als $C_1$—$C_6$-Alkylgruppe, $C_1$—$C_6$-Alkoxygruppe, Nitrogruppe, $C_1$—$C_6$-Alkylmercaptogruppe, Methylendioxygruppe, Chlor, Brom, Jod, Fluor oder Trifluormethylgruppe definiert ist.

2. Verbinding nach Anspruch 1, dadurch gekennzeichnet, daß $R_1$ eine Ethylgruppe ist und die Verbindung (−)N-Ethyl-2,10,11-trihydroxynoraporphin ist.

3. Verbindung nach Anspruch 1, dadurch gekennzeichnet, daß $R_1$ eine n-Propylgruppe ist und die Verbindung (−)N-n-Propyl-2,10,11-trihydroxynoraporphin ist.

4. Verbindung nach Anspruch 1, dadurch gekennzeichnet, daß $R_3$ eine Methylgruppe ist und die Verbindung (−)N-n-Propyl-2,10,11-triacetoxynoraporphin ist.

5. Verbinding nach Anspruch 1, dadurch gekennzeichnet, daß $R_1$ eine Allylgruppe ist und die Verbindung (−)N-Allyl-2,10,11-trihydroxynoraporphin ist.

6. Verbindung nach Anspruch 1, dadurch gekennzeichnet, daß $R_1$ eine 2′-Bromallylgruppe ist und die Verbindung N-(2′-Bromallyl)-2,10,11-trihydroxynoraporphin ist.

7. Verbindung nach Anspruch 1, dadurch gekennzeichnet, daß $R_1$ eine 2-Chlorethylgruppe ist, $R_2$ Wasserstoff oder die Gruppe

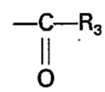

ist, $R_3$ eine $C_1$—$C_6$-Alkylgruppe ist, $R_4$ Wasserstoff ist, sowie deren Säureadditionssalze.

8. Verbinding nach Anspruch 7, dadurch gekennzeichnet, daß sie aus (−)N-(2-Chlorethyl)norapomorphin besteht.

**Revendications**

1. Un composé de formule

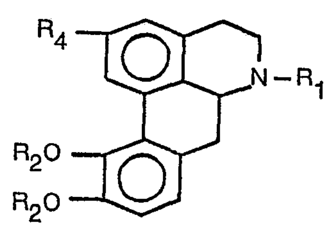

dans laquelle $R_1$ représente un groupe alkyle en $C_1$—$C_6$, alkyle en $C_1$—$C_6$ substitué, cycloalkyle en $C_3$—$C_8$, cycloalkyle en $C_3$—$C_8$ substitué, alcényle en $C_3$—$C_7$, alcynyle en $C_3$—$C_7$, alcynyle en $C_3$—$C_7$ substitué, phényl-alkyle en $C_1$—$C_4$ éventuellement substitué, phényl-alcényle en $C_2$—$C_4$ éventuellement substitué at phényl-alcynyle en $C_2$—$C_4$ éventuellement substitué, $R_2$ représente l'hydrogène ou un groupe

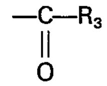

dans laquel $R_3$ représente un groupe alkyle en $C_1$—$C_7$, $R_4$ représente l'hydrogène ou un groupe $R_2O$, et ses sels formés par addition avec des acides, avec la restriction que lorsque $R_4$ représente l'hydrogène, $R_1$ représente un groupe 2-chloroéthyle, l'expression "substitué" se référant à des groupes alkyles en $C_1$—$C_6$, alcoxy en $C_1$—$C_6$, nitro, alkylmercapto en $C_1$—$C_6$, méthylène-dioxy, le chlore, le brome, l'iode, le fluore ou des groupes trifluorométhyle.

2. Un composé selon la revendication 1, dans lequel $R_1$ représente un groupe éthyle, ledit composé étant la (−)N-éthyl-2,10,11-trihydroxynoraporphine.

3. Un composé selon la revendication 1, dans lequel $R_1$ représente un groupe n-propyle, ledit composé étant la (−)N-n-propyl-2,10,11-trihydroxynoraporphine.

4. Un composé selon la revendication 1, dans lequel $R_3$ représente un groupe méthyle, ledit composé étant la (−)N-n-propyl-2,10,11-triacétoxynoraporphine.

5. Un composé selon la revendication 1, dans lequel $R_1$ représente un groupe allyle, ledit composé étant la (−)N-allyl-2,10,11-trihydroxynoraporphine.

6. Un composé selon la revendication 1, dans lequel $R_1$ représente un groupe 2'-bromoallyle, ledit composé étant la N-(2'-bromoallyl)-2,10,11-trihydroxynoraporphine.

7. Le composé selon la revendication 1, dans lequel $R_1$ représente un groupe 2-chloroéthyle, $R_2$ l'hydrogène ou le groupe

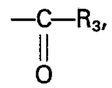

$R_3$ représente un groupe alkyle en $C_1$—$C_6$, $R_4$ représente l'hydrogène, et ses sels formés par addition avec des acides.

8. Un composé selon la revendication 7, consistant en la (−)N-(2-chloroéthyl)norapomorphine.

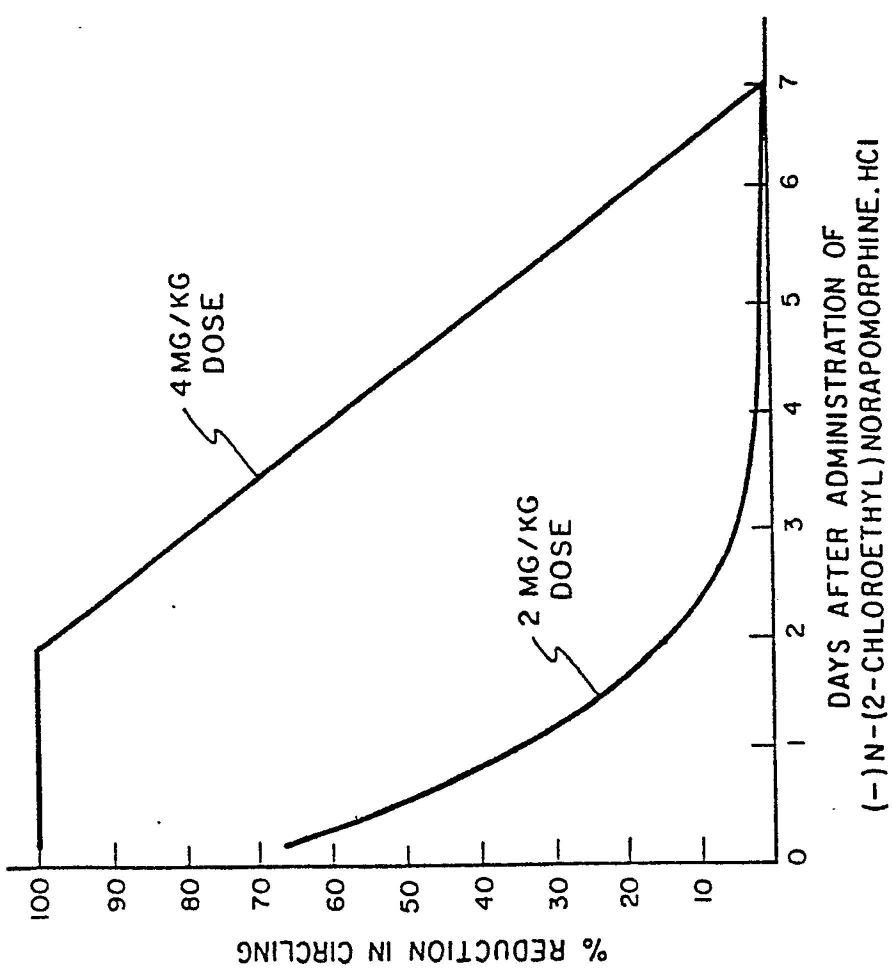


INHIBITION OF CIRCLING IN MICE PREVIOUSLY CHALLENGED WITH APOMORPHINE

Fig.1.

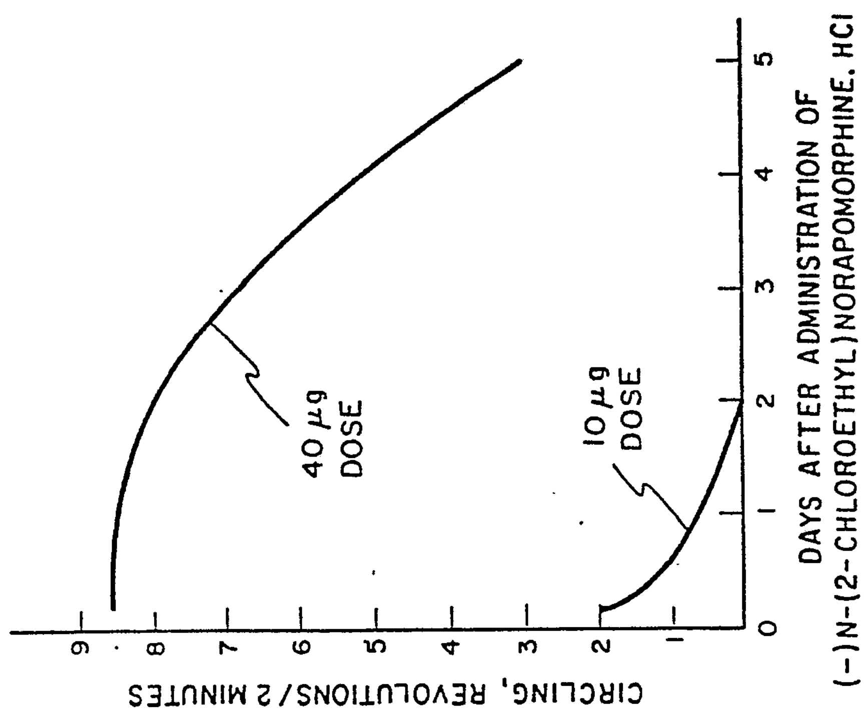


INDUCTION OF CIRCLING IN RATS

Fig.2.

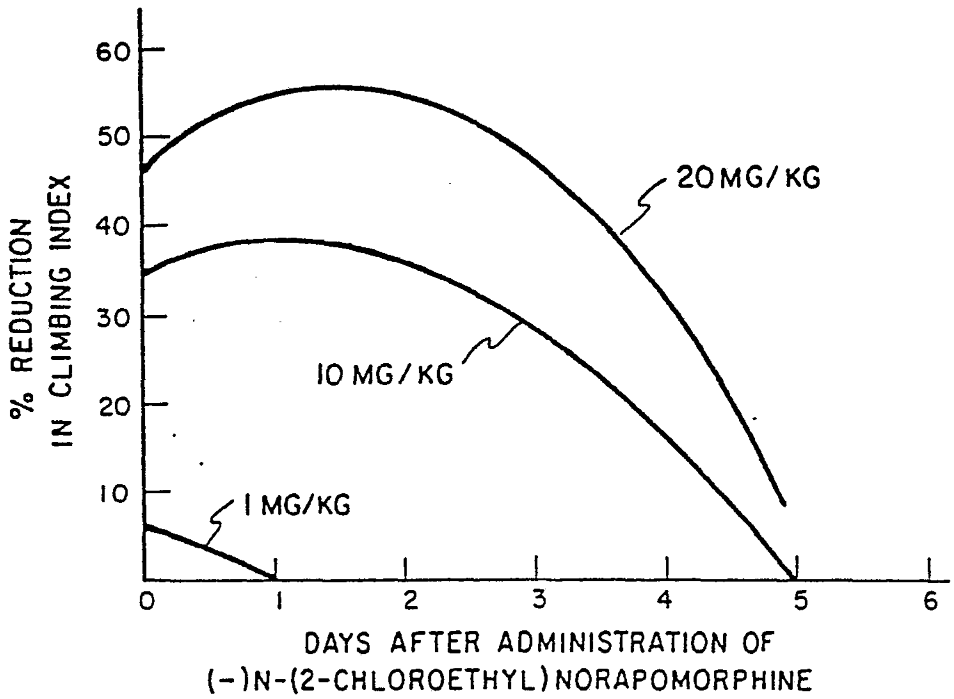


INHIBITION OF CLIMBING IN MICE PREVIOUSLY CHALLENGED WITH APOMORPHINE

*Fig.3.*

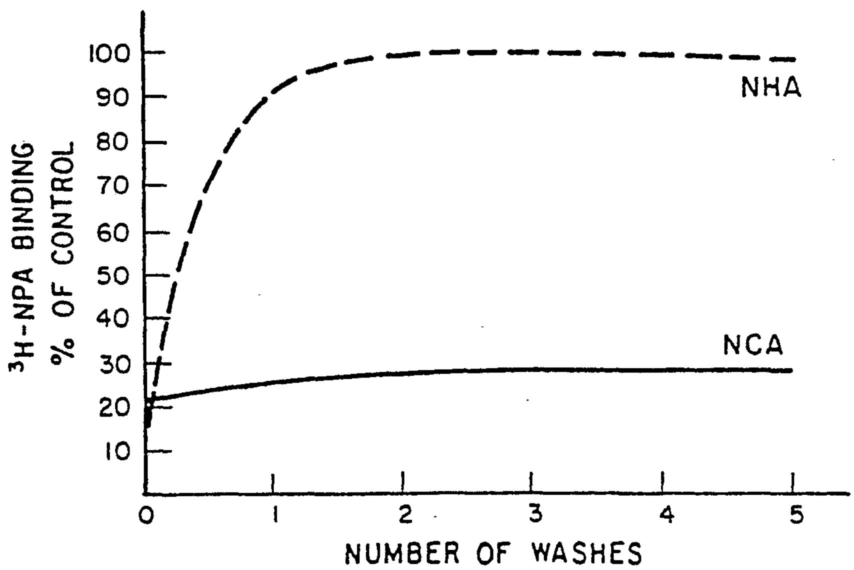


EFFECT OF WASHING ON THE BINDING OF -)N-(2-CHLOROETHYL)NORAPOMORPHINE. HCl (NCA) AND OF (-)N-(2-HYDROXYETHYL)NORAPOMORPHINE. HCl (NHA) TO RAT STRIATAL HOMOGENATE

*Fig.7.*

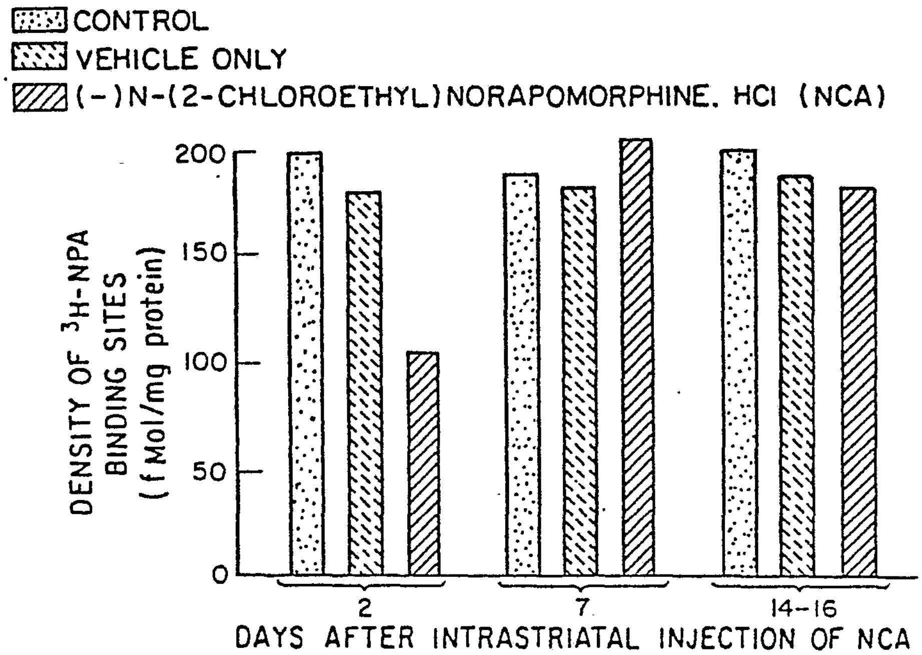


IN VIVO EFFECT OF THE INTRASTRIATAL BINDING OF (N)-(2-CHLOROETHYL)NORAPOMORPHINE. HCl ON SUBSEQUENT BINDING OF $^3$H-N-n-PROPYLNORAPOMORPHINE ($^3$H-NPA)

*Fig.4.*

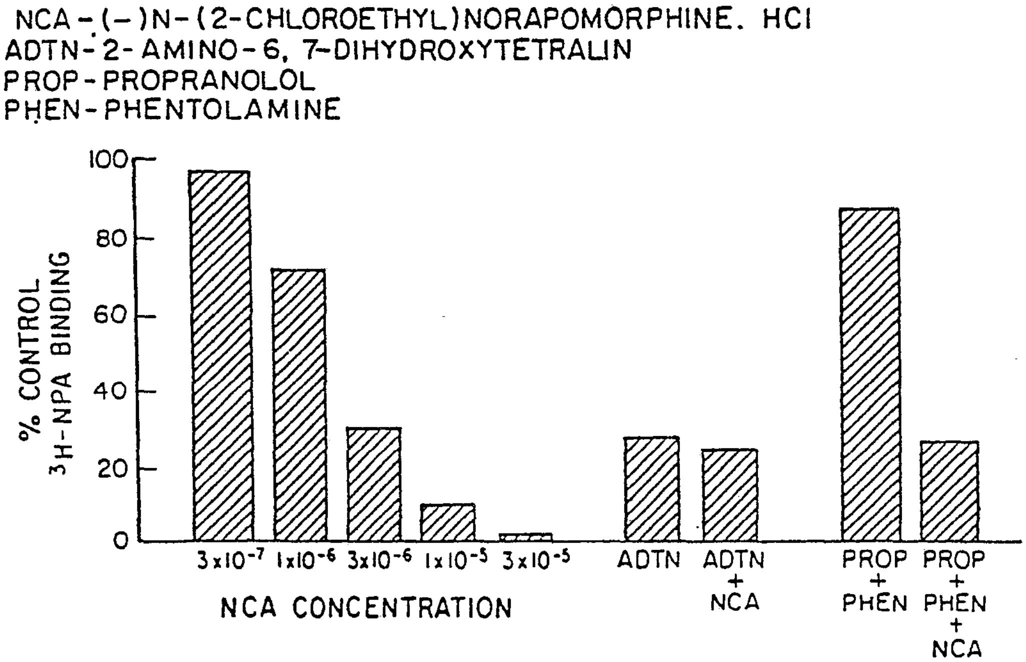


IN VITRO EFFECT OF THE INTRASTRIATAL BINDING OF (N)-(2-CHLOROETHYL)NORAPOMORPHINE, HCl ON SUBSEQUENT BINDING OF $^3$H-N-n-PROPYLNORAPOMORPHINE ($^3$H-NPA) ALONE OR IN PRESENCE OF OTHER BLOCKING AGENTS

*Fig.5.*

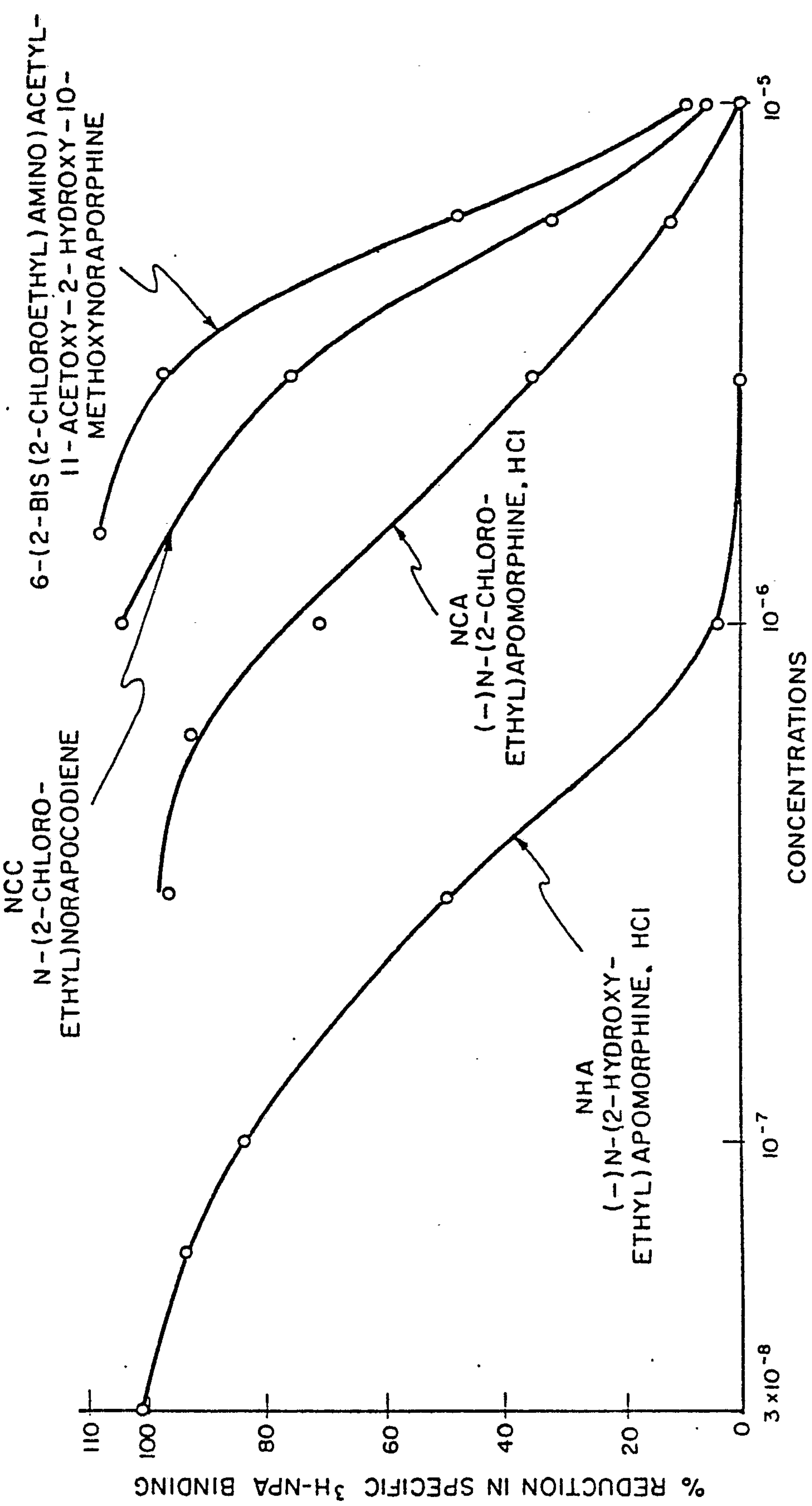


ABILITY OF DIFFERENT APOMORPHINE-TYPE COMPOUNDS TO REDUCE THE SPECIFIC BINDING OF $^3$H-N-n-PROPYLNORAPOMORPHINE IN RAT STRIATAL HOMOGENATE

*Fig.6*

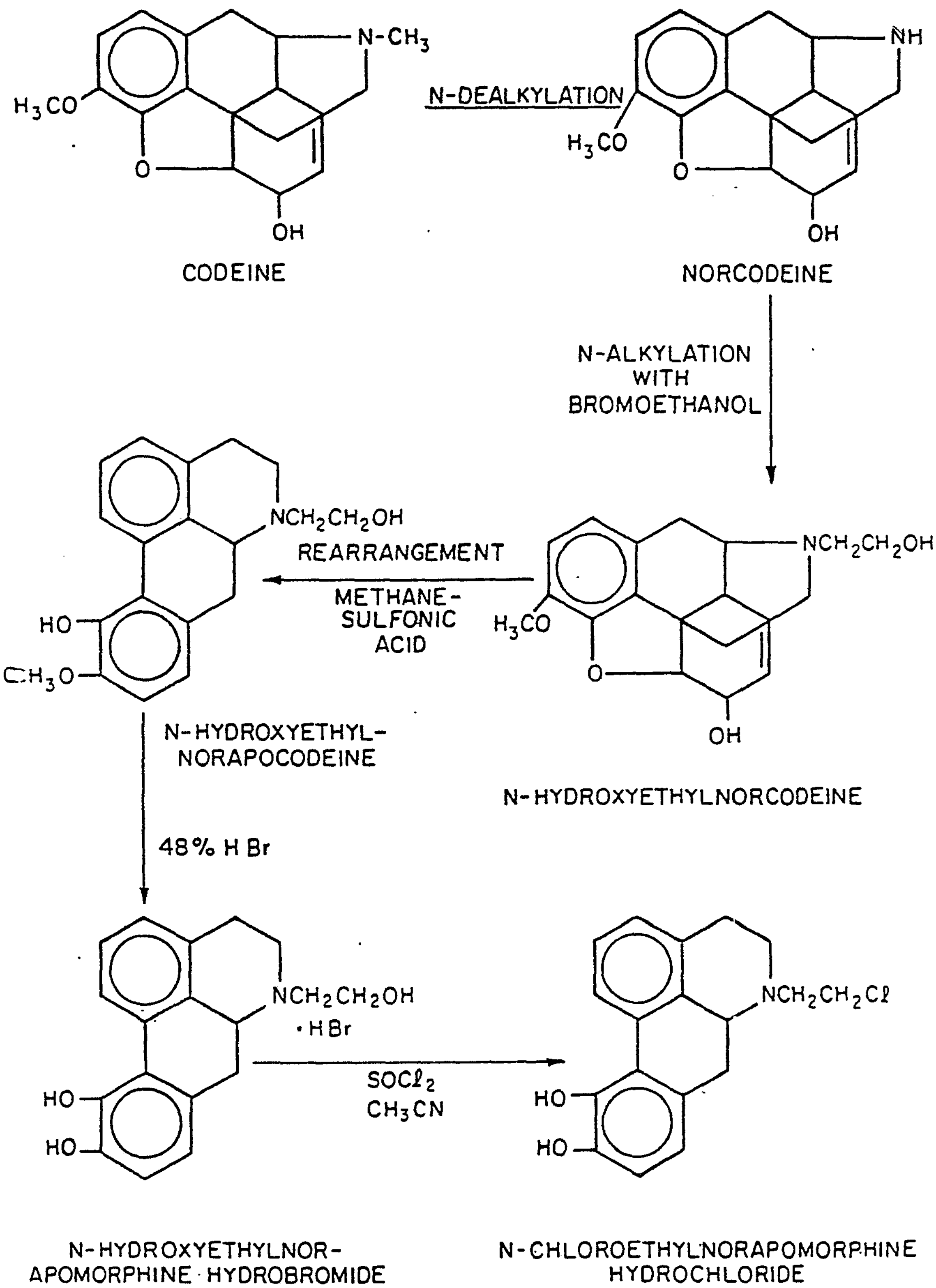


CODEINE

NORCODEINE

N-HYDROXYETHYLNORCODEINE

N-HYDROXYETHYL-NORAPOCODEINE

N-HYDROXYETHYLNOR-APOMORPHINE·HYDROBROMIDE

N-CHLOROETHYLNORAPOMORPHINE HYDROCHLORIDE

*Fig.8.*